# EUROPEAN PATENT APPLICATION

(11) **EP 4 631 966 A1**
(43) Date of publication of application: **15.10.2025**
(21) Application number: 23900909.5
(22) Date of filing: 02.11.2023
(51) Int. Cl.: C07K 7/06, A61P 1/02, A23L 33/18, A61K 38/00

(54) **NOVEL PEPTIDE**

(30) Priority: 08.12.2022 KR 20220170847
(71) Applicant: Hysensbio Co., Ltd, Gwacheon-si Gyeonggi-do 13814 (KR)
(72) Inventor: PARK, Joo Hwang, Incheon 21986 (KR); LEE, Dong Seol, Seoul 03088 (KR); LEE, Ji Hyun, Seoul 03088 (KR)
(74) Representative: Mewburn Ellis LLP
(86) International application number: PCT/KR2023/017402
(87) International publication number: WO 2024/122889

(57) **Abstract**

The present invention relates to a novel peptide, a polynucleotide encoding the peptide, an expression vector including the polynucleotide, and a pharmaceutical composition, quasi-drug composition, and health functional food composition including the peptide.

## Description

### [Technical Field]

The present invention relates to a novel peptide, more specifically to a peptide for promoting regeneration of a hard tissue and/or a dental pulp tissue and for treating the dentin-dental pulp disease and/or the periodontal disease, a polynucleotide encoding the peptide, an expression vector including the polynucleotide, a pharmaceutical composition for preventing or treating the dentin-dental pulp disease and/or the periodontal disease including the peptide, a quasi-drug composition for prevention or improvement of the dentin-dental pulp disease and/or the periodontal disease, and a health functional food composition for prevention or improvement.

### [Background Art]

The dental pulp is a soft connective tissue that fills a dental pulp cavity inside the tooth and is richly distributed with nerves and blood vessels, and refers to a place that reaches a surface layer of dentin. Diseases of the dental pulp are called dental pulp diseases.

The causes of dental pulp disease are very diverse, but most cases are caused by bacterial infection caused by tooth decay and infection into the pulp through perforation, fracture, fissure, and periodontal pocket of the tooth. It can also cause trauma, abrasion, tooth cracking, and heat and friction from dental instruments during treatment. Pulpitis caused by bacterial infection can extend to root apex and periodontal diseases. When dental pulp disease occurs, it progresses in the order of dental pulp congestion, pulpitis, and dental pulp necrosis. In the case of dental pulp necrosis, since the dental pulp dies and the blood supply to the dental pulp is completely lost, the entire periodontal tissue disappears, resulting in the root apex disease or abnormality of the entire tooth later.

Treatment of dental pulp and root apex diseases uses dental pulp replicating agents and dental root canal filling materials, and calcium hydroxide, mineral trioxide aggregate (MTA), Gutta-Percha, and the like have been generally used. MTA has a sealing force and biocompatible properties, so it is effective in treatment, but a relatively high-cost problem occurs as a dental treatment, and aesthetic problems occur due to discoloration. In the case of Gutta-Percha, the cost is economical, and the liquidity is good, but it is an unphysiological treatment method that loses dental pulp vitality. Until now, conservative treatment methods for treating a dentin-dental pulp disease cause the treated tooth to become weak, brittle, and have a risk of re-infection.

A periodontal tissue (periodontium) is a complex organ composed of epithelial tissue, a soft connective tissue, and calcified connective tissue. Structures of the periodontal tissue include gingiva, periodontal ligament (PDL), cementum, and alveolar bone. Gingival fibroblasts and periodontal ligament fibroblasts are major cell components of gingival soft tissue connective tissue and play a role in forming and maintaining an extracellular matrix. Among them, it is known that gingival fibroblasts are mainly involved in maintaining a gingiva connective tissue, whereas the periodontal ligament fibroblasts are involved in the restoration and regeneration of adjacent alveolar bone and cementum in vivo as well as forming periodontal ligament with their unique function. A periodontal disease clinically results in tooth loss due to gingiva bleeding and swelling formation of periodontal pockets, and destruction of alveolar bone.

Since the ultimate result of treating the periodontal disease is to restore damaged connective tissue, cementum, and alveolar bone, to this end, not only the periodontal ligament supporting the alveolar bone needs to be regenerated but also the alveolar bone and cementum to which the periodontal ligament can be attached need to be regenerated.

Accordingly, research is actively progressing to develop a therapeutic agent capable of effectively treating the dentin- dental pulp disease. For example, Korean Patent Publication No. 2012-0089547 discloses a composition for hard tissue formation and dentin or dental pulp tissue regeneration, including ameloblasts, apical bird cells, or a culture medium thereof as an active ingredient, and Korean Patent Publication No. No. 2009-0033643 discloses a new dental folliclederived dental stem cell and a method for culturing the same. In addition, Korean Patent Publication No. 2016-0105627 discloses a composition for treating periodontal disease comprising an ameloblast culture medium.

Under this background, in order to develop an agent that can more effectively treat the dentin-pulpal disease and/or the periodontal disease causing alveolar bone and cementum damage, as a result of intensive research efforts, the present inventors have developed cell therapy for promoting regeneration of the hard tissue including dentin, bone and cementum and/or the dental pulp tissue, and peptides exhibiting therapeutic effects for the dentin-dental pulp disease and/or periodontal disease, and the present invention completed.

### [Disclosure]

### [Technical Problem]

An object of the present invention is to provide a peptide for promoting the regeneration of a hard tissue and/or a dental pulp tissue and for treating a dentin-dental pulp disease and/or periodontal disease.

Another object of the present disclosure is to provide a polynucleotide encoding the peptide.

Another object of the present disclosure is to provide an expression vector, including the polynucleotide.

Another object of the present invention is to provide a pharmaceutical composition for preventing or treating dentin-dental pulp disease and/or periodontal disease, including peptide.

Another object of the present invention is to provide a quasi-drug composition for preventing or improving the dentin-dental pulp disease and/or periodontal disease, including the peptide.

Another object of the present invention is to provide a health functional food composition for preventing or improving dentin-dental pulp disease and/or periodontal disease, including the peptide.

Another object of the present invention is to provide a method for preventing or treating dentin-dental pulp disease and/or periodontal disease in a non-human subject, including administering a composition including the peptide to the subject.

Another object of the present invention is to provide a method for promoting the regeneration of a hard tissue, including dentin, bone, and cementum, or a dental pulp tissue in a non-human subject, including administering a composition including the peptide to the subject.

The objects of the present invention are not limited to those mentioned above, and further objects not mentioned will be apparent to those skilled in the art from the following description.

### [Technical Solution]

According to one aspect of the present invention for solving the above technical problem, a peptide for promoting regeneration of a hard tissue and/or a dental pulp tissue and treating a dentin-dental pulp disease and/or a periodontal disease, including an amino acid sequence of General formula 1 below is provided:

K-Y-R1-R2-R3-R4-R5-R6-Y-K (General formula 1)

In General, formula 1, R1, and R2 are each lysine (K), alanine (A) or arginine (R), R3, R4, and R5 are each lysine (K) or arginine (R), and R6 is asparagine (N) or serine (S).

According to one embodiment, the peptide may be composed of any one of amino acid sequences of SEQ ID NOs: 1 to 64.

According to one embodiment, the peptide may be composed of any one of an amino acid sequences of SEQ ID NOs: 1 to 8.

According to one embodiment, the peptide may be subjected to N-terminal or C-terminal acetylation, amidation, or methylation; introduction of D-amino acid; peptide bond modification such as CH₂-NH, CH₂-S, CH₂-S=O, or CH₂-CH₂; backbone modification; or side chain modification.

According to one embodiment, the hard tissue may include dentin, bone, and cementum.

As another aspect, the present invention provides a polynucleotide encoding the peptide.

As another aspect, the present invention provides an expression vector, including the polynucleotide.

As another aspect, the present invention provides a pharmaceutical composition for preventing or treating a dentin-dental pulp disease, including the peptide.

According to one embodiment, the dentin-dental pulp disease may be dentine hyperesthesia, pulp hyperemia, pulpitis, pulp modification, pulp necrosis, and gangrene.

As another aspect, the present invention provides a pharmaceutical composition for preventing or treating periodontal disease, including peptide.

According to one embodiment, periodontal disease may be gingivitis, periodontitis, periodontal pocket, or periodontal abscess.

As another aspect, the present invention provides a quasi-drug composition for preventing or improving the dentin-dental pulp disease and/or periodontal disease, including the peptide.

As another aspect, the present invention provides a health functional food composition for preventing or improving dentin-dental pulp disease and/or periodontal disease, including the peptide.

As another aspect, the present invention provides a method for preventing or treating a dentin-dental pulp disease in a non-human subject, including administering a composition including the peptide to the subject.

As another aspect, the present invention provides a method for preventing or treating periodontal disease in a non-human subject, including administering a composition including the peptide to the subject.

As another aspect, the present invention provides a method for promoting the regeneration of hard tissue, including dentin, bone, and cementum, or dental pulp tissue in a non-human subject, including administering a composition including the peptide to the subject.

### [Advantageous Effects]

Since a peptide for promoting regeneration of a hard tissue and/or a dental pulp tissue and for treating a dentin-dental pulp disease and/or a periodontal disease of the present invention exhibits an excellent regeneration promoting effect of the hard tissue and/or the pulp tissue, it will be widely used in the development of preparations for preventing or treating various dentin-dental pulp diseases, or in the development of preparations for preventing or treating the periodontal diseases that cause bone and/or cementum damage.

The effects of the present invention are not limited to those mentioned above, and other effects that are not mentioned may be clearly understood by those skilled in the art to which the present invention pertains, from the description below.

### [Description of Drawings]

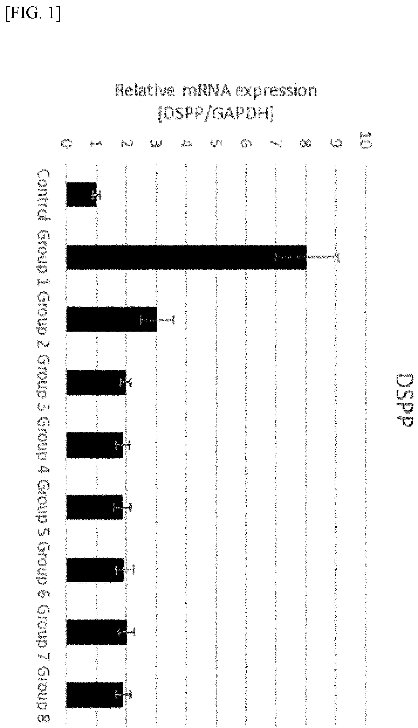

FIG. 1 shows a result showing the effect of each group of peptides on the expression of dentin sialophosphoprotein (DSPP), which is a marker gene for odontoblast differentiation in human dental pulp cells by grouping a new peptide in which an amino acid sequence is substituted (as an average value of each group measured by quantitative real-time PCR for a level of DSPP mRNA in human dental pulp cells, the peptide was treated at a concentration of 10 ug/ml).

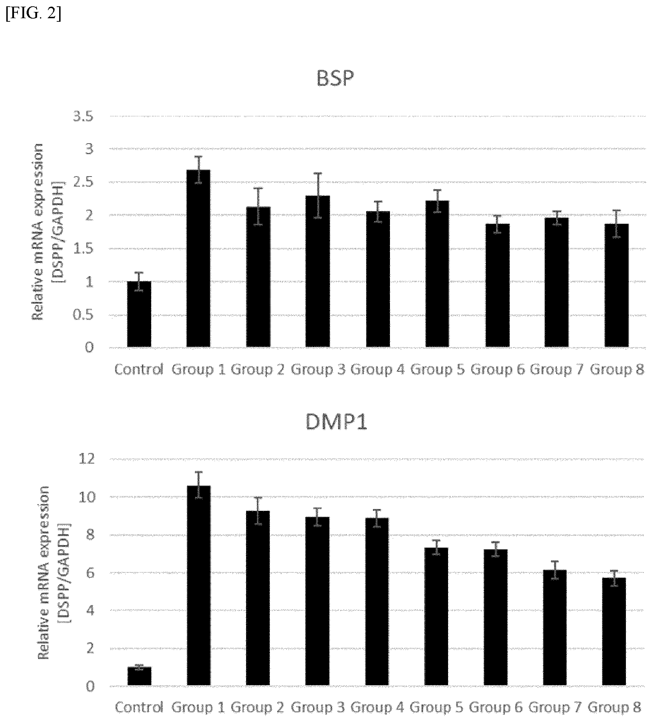

FIG. 2 shows the result of confirming the BSP and DMP1 gene expression by real-time PCR after treating the peptides of each group in human-derived mesenchymal stem cells in order to confirm the effect of the novel peptide on the expression of BSP and DMP1 genes, which are marker genes for differentiation of osteoblasts and cementoblasts.

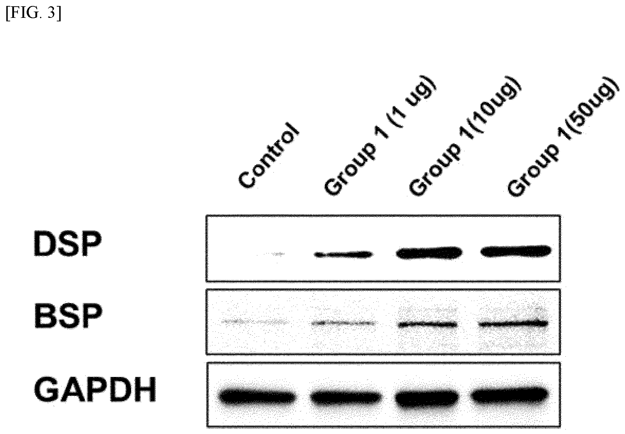

FIG. 3 is a result of confirming expressions of DSP and BSP proteins by Western blot after treating human dental pulp cells with the novel peptide (SEQ ID NO: 1) in a concentration-dependent manner (1, 10, and 50 ug) to confirm the effect of novel peptides on expressions of marker proteins for differentiation of odontoblasts, osteoblasts, and cementoblasts.

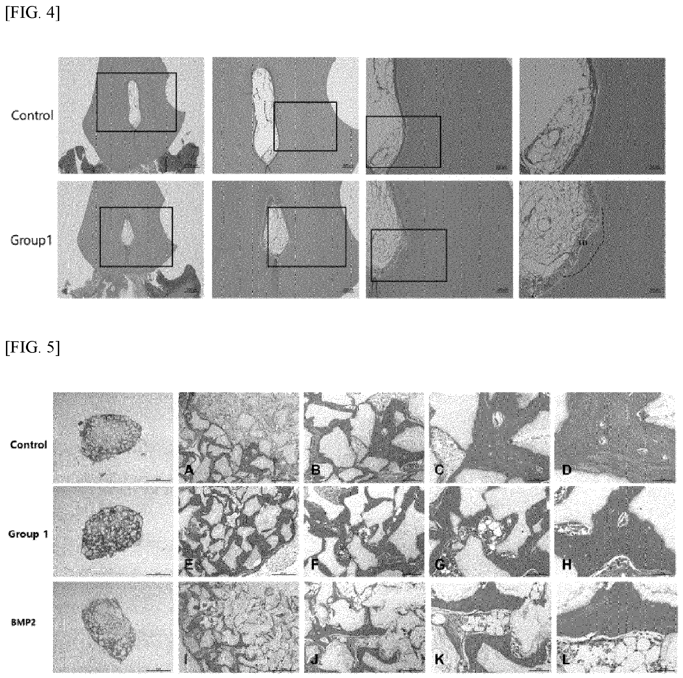

FIG. 4 is the result of histological evaluation 3 weeks after the application of 10 ug of the novel peptide (SEQ ID NO: 1) after damaging the dentine of beagle dog's teeth in order to confirm that the novel peptide induces physiological dentin regeneration in an animal model of damaged dentin.

Based on the in vitro test results shown in FIG. 2 and Tables 10 and 11, in order to confirm the effect of the peptide of Group 1 (SEQ ID NO: 1) on hard tissue formation in vivo, FIG. 5 shows the result confirming that SEQ ID NO: 1 peptide (10 ug) of group 1 or human dental pulp cells (hDPCs) of control (cell only) were transplanted into the subcutaneous tissue of mice with impaired immune systems, and the proportion of hard tissue formation was increased in the group treated with the novel peptide compared to the control group 12 weeks after transplantation (A-D: hDPCs-only; E-H: group 1 peptide; I-L: BMP2 treatment/size bar; A, E, and I: 500 µm; B, F, J: 200 µm; C, G, and K: 100 µm; D, H, and L: 50 µm)

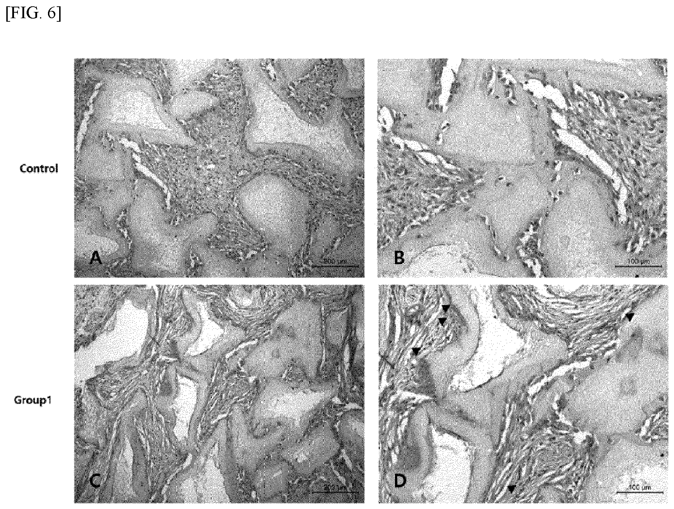

In order to confirm the effect of Group 1 (SEQ ID NO: 1) peptides on periodontal ligament formation in vivo, FIG. 6 shows the result confirming that SEQ ID NO: 1 peptide (10 ug) of group 1 or human dental pulp cells (hDPCs) of control (cell only) were transplanted into the subcutaneous tissue of mice with impaired immune systems, and compared to the control group 6 weeks after transplantation (size bar; A, C: 200 µm; B and D: 100 µm).

### [Modes of the Invention]

Objects and advantages of the present invention and technical configurations for achieving them will become apparent with reference to the embodiments described in detail below in conjunction with the accompanying drawings. In the description of the present invention, when it is determined that a specific description of a known function or configuration may unnecessarily obscure the gist of the present invention, the detailed description thereof will be omitted. And the following terms are defined terms in consideration of the donation in the present invention, which may vary according to the user, the intent or practice of the operator, etc.

However, the present invention is not limited to the embodiments disclosed below but may be embodied in various different forms. The examples are merely provided to complete the present invention's disclosure and fully illuminate the invention's scope to those skilled in the art, and the scope of the claims only defines the invention. The definition should therefore be made on the basis of the disclosure throughout this specification.

Hereinafter, the present invention will be described in detail.

The inventors of the present invention developed a novel peptide composed of 10 amino acids as a result of various studies to develop preparation capable of more effectively treating a dentin-dental pulp disease and/or periodontal disease.

The novel peptide developed above is prepared by partially substituting an amino acid sequence of a peptide capable of exhibiting a therapeutic effect for the dentin-dental pulp disease and/or the periodontal disease since the peptide can increase an expression level of the DSPP gene, which is a marker gene for odontoblast differentiation, the peptide exhibits an effect of promoting dentin regeneration, and it is possible to increase the expression levels of BSP (Bone sialoprotein) gene and DMP1, which are marker genes of differentiation for osteoblasts and cementoblasts, so that bone and cementum regeneration can be promoted.

In addition, it was confirmed that a transplant including the peptide together with human dental pulp cells was prepared, and the transplant was transplanted into the subcutaneous tissue of a damaged mouse in an immune system, after 6 weeks or 12 weeks, the transplanted tissue was analyzed and, as a result, a dentin-pulp similar tissue in the form most similar to the dentin-pulp tissue in vivo was formed, the bone-like tissue in the form most similar to the bone tissue in vivo is formed, the level of collagen formation increases, and an expression level of DSP which is an odontoblast-specific differentiation marker gene is increased.

In addition, as a result of analyzing the shape of the transplanted tissue using a scanning electron microscope, it was confirmed that odontoblast-like cells were observed along the formed hard tissue and that odontoblast projections also extended toward the formed hard tissue. In addition, it was confirmed that the surface of the formed hard tissue exhibits characteristics of typical osteoblasts and/or cementoblasts in which cuboidal cells are attached.

Therefore, it was found that the peptide of the present invention can promote the regeneration of the hard tissue and/or the pulp tissue and exhibit effects on the dentin-dental pulp disease and/or periodontal disease. The peptide of the present invention exhibiting such an effect has not been reported at all so far and was developed for the first time by the present inventors.

As used herein, the term "hard tissue" refers to relatively hard skeletal tissue including bone, hyaline cartilage, and fibrous cartilage. In one embodiment according to the present invention, the hard tissue may include dentin, bone, and cementum.

The term of the present invention, "dentine" is also called "tooth matter", it means a hard tissue of yellowish white, constituting most of the tooth. The dentin is covered with enamel in the crown and cementum in the root so that it is not exposed to the surface of the tooth, but as the age increases, when the enamel wears away, the dentin may be exposed to the tip of the crown or an occlusal surface. Although dentin is a kind of bone-like tissue, it is distinguished from general bone tissue in that the main body of the cells making the dentin is in the tooth pulp, and only its protrusion extends into the dentin.

The term "cementum" of the present invention refers to a thin film in the form of a slightly deformed bone covering the tooth roots and other parts of mammals. The cementum is composed of 50% inorganic matter and 50% water-organic matter, has a yellow color and has a lower hardness than dentin or enamel. The cementum includes periodontal ligament fibers that fix the teeth to an alveolar bone, and when bacteria are infected in the gums, the cementum surrounding the teeth is modified, and the periodontal ligament fibers that connect the teeth and the alveolar bone do not stick to the modified cementum so that the teeth become loose. In order to treat this modification of the cementum, a method of removing the modified cementum and promoting the formation of new cementum has been used.

The peptide provided in the present invention can increase the expression levels of the DSPP gene, which is a marker gene for odontoblast differentiation, and the BSP and DMP1 genes which are marker genes for differentiation of osteoblasts and cementoblasts, and When transplanted into a living body together with human dental pulp cells, the human dental pulp cells can exhibit characteristics of forming dentin/pulp tissue-like tissue and bone-like tissue.

In the peptide provided in the present invention, variant peptides having a sequence in which one or more amino acid residues differ from the amino acid sequence constituting the peptide are also included in the scope of the peptides provided by the present invention, as long as it can show the effect of promoting regeneration of the hard tissue including dentin, bone and cementum and treating the dentin-pulp disease and/or the periodontal disease.

In general, amino acid exchanges in proteins and polypeptides that do not entirely alter the activity of the molecule are known in the art. The most commonly occurring exchange is the exchange between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly. In addition, peptides with increased structural stability against heat, pH, and the like, or increased regeneration-promoting ability of a hard tissue including dentin, bone and cementum and/or a dental pulp tissue may be included by mutation or modification in an amino acid sequence.

Amino acid variations are made based on the relative similarity of amino acid side chain substituents, such as hydrophobicity, hydrophilicity, charge, size, and the like. Among the amino acids constituting the peptides of the present invention, asparagine (N) and serine (S) correspond to a hydrophilic amino acid, so that the amino acid side chain substituents have high relative similarity. Accordingly, even if the amino acid constituting the peptide, according to the embodiment of the present invention are substituted with a hydrophilic amino acid, the amino acid may exhibit similar effects to each other due to their structural similarity.

For example, asparagine which is an acidic amino acid located at position 8 of the peptide of SEQ ID NO: 1 provided in the present invention, can exhibit the effect of the peptide provided in the present invention even if it is substituted with serine, and Even if lysine which is a basic amino acid located at position 5 of the peptide of SEQ ID NO: 1, is substituted with arginine which is a basic amino acid, the effect of the peptide provided in the present invention can be exhibited.

Even when the acidic amino acids or basic amino acids constituting the peptide of the present invention are substituted with another acidic amino acids or basic amino acids, the effect of the peptide provided in the present invention can be exhibited as it is, since the effects of the peptide provided in the present invention can be exhibited as they are, it is obvious that a variant peptide having a sequence that partially differs from the amino acid sequence constituting the peptide of the present invention by at least one amino acid residue are also included in the scope of the peptides provided in the present invention.

In addition, even when the peptide of the present invention has a form in which any amino acid is added to N-terminus or C-terminus thereof, an effect of the peptide provided in the present invention can be shown as it is, so that it falls within the scope of the peptide provided in the present invention. As an example, it may be in a form in which 1 to 300 amino acids are added to an N-terminus or C-terminus of the peptide, as another example, it may be in a form in which 1 to 100 amino acids are added to the N-terminus or C-terminus of the peptide, and as another example, it may be in a form in which 1 to 24 amino acids are added to the N-terminus or C-terminus of the peptide.

In order to protect from proteolytic enzymes in vivo and increase stability, the peptide of the present invention may be a modified form in which the N-terminus and/or C-terminus thereof are chemically modified or protected with an organic group, or an amino acid is added to the peptide terminus. In particular, in the case of chemically synthesized peptides, since the N-terminus and C-terminus are charged, in order to remove this charge, acetylation of N-terminus, methylation of N-terminus, and/or amidation of C-terminus may be performed, or introduction of D-amino acid, peptide bond modification such as CH₂-NH, CH₂-S, CH₂S=O, and CH₂-CH₂, backbone modification, side chain modification may be performed, but is not limited thereto. Methods for preparing peptide mimetic compounds are known in the art. See for example Quantitative Drug Design(C.A. Ramsden Gd., Choplin Pergamon Press (1992)).

The term "backbone modification" of the present invention refers to a chain or ring-shaped backbone that is the main amino acid constituting the peptide, and
direct modification of amino acids constituting these peptide backbones into amino acid analogs is called backbone modification. The amino acid analog refers to an amino acid modified by substitution of a hydrogen atom at nitrogen or α-carbon of the amino acid backbone.

The term "side chain modification" of the present invention refers to an atomic group branched like a branch from a chain or ring-shaped backbone that is the main amino acid constituting the peptide as a side chain of the amino acid, and modifying these side chains using chemicals is called side chain modification. Examples of peptide side chain modifications include reductive alkylation reactions; amidylation by methylacetimidate; alkylation by acetic anhydride; carbamorylation of an amino group by cyanate; trinitrobenzylation of an amino acid by 2,4,6-trinitrobenzene sulfonic acid (TNBS); alkylation of an amino group by succinic anhydride; or modification of an amino group such as pyridosylation reduced with NaBH₄ after treatment with pyridoxal-5phosphate.

In addition, the peptide of the present invention may be used alone, but may be used in combination with a pharmaceutically approved carrier such as an organic solvent, and the peptide may be used including carbohydrates such as glucose, sucrose or dextran, antioxidants such as ascorbic acid or glutathione, chelating agents, small molecular proteins, and other stabilizers to increase stability and efficacy.

According to one embodiment of the present invention, 64 kinds of peptides corresponding to general General formula 1 provided in the present invention were synthesized, and the effect of the synthesized peptides on the expression level of a DSPP gene which is a marker gene for odontoblast differentiation, was verified. As a result, it was confirmed that the mRNA level of the DSPP gene in human dental pulp cells treated with the 64 peptides exhibited a value of 9.7 fold or higher, 6 fold or higher, 3 fold or higher, or at least about 1.5 fold or higher, compared to the mRNA level of the DSPP gene which is a differentiation marker of odontoblasts measured in human dental pulp cells (control group) not treated with the peptide of the present invention (FIG. 1 and Table 10).

According to what has been reported so far, Since it is known that when the mRNA expression level of DSPP is increased, odontoblast differentiation and dentin regeneration are promoted, the 64 peptides exhibiting the effect of increasing the mRNA level of the DSPP gene promote odontoblast differentiation and dentin regeneration (Taduru Sreenath et al., THE JOURNAL OF BIOLOGICAL CHEMISTRY, Vol. 278, No. 27, Issue of July 4, pp. 24874724880, 2003; William T. Butler et al, Connective Tissue Research, 44(Suppl. 1): 171178, 2003).

In addition, the effect of the synthesized peptide on the expression level of the BSP gene, which is a marker gene for differentiation of osteoblasts/cementoblasts, was verified. As a result, it was confirmed that the mRNA level of the BSP gene in human dental pulp cells treated with the 64 peptides exhibits 2.9-fold or higher, or at least about 1.6-fold or higher, and the mRNA level of the DMP1 gene exhibits a value of 10.6-fold or higher, or at least about 5.7-fold or higher, compared to the mRNA level of the DSPP gene which is a marker gene for differentiation of osteoblasts/cementoblasts measured in human dental pulp cells (control group) not treated with the peptide of the present invention (Fig. 2 and Table 11, Table 12).

Accordingly, since it is known that when the mRNA expression level of BSP is increased, differentiation of osteoblasts/cementoblasts and regeneration of bone and cementum are promoted, the 64 peptides exhibiting the effect of increasing the mRNA level of the BSP gene exhibit the effect of promoting differentiation of osteoblasts/cementoblasts and regeneration of bone and cementum.

As another aspect, the present invention provides a polynucleotide encoding the peptide.

The polynucleotide may be mutated by substitution, deletion, insertion, or a combination of one or more bases. When preparing a nucleotide sequence by chemical synthesis, a synthesis method well known in the art, for example, a method described in a document (Engels and Uhlmann, Angew Chem IntEd Engl., 37:73-127, 1988), triester, phosphite, phosphoramidite and H-phosphate methods, PCR and other auto-primer methods, and an oligonucleotide synthesis method on a solid support can be used.

As another aspect, the present invention provides an expression vector including the polynucleotide, a transformant including the expression vector, and a method for preparing the peptide using the transformant.

The term "expression vector" of the present invention means a recombinant vector capable of expressing peptide of interest in a host cell of interest, which gene construct includes essential regulatory elements operably linked to express a gene insert. The expression vector includes expression control elements, such as initiation codon, termination codon, promoter, operator, etc., which are generally considered to be part of a nucleotide sequence encoding a polypeptide and must be functional in an individual when the genetic construct is administered and in frame with the coding sequence. The promoter of the vector may be constitutive or inducible.

The term "operably linked" of the present invention refers to a state in which a nucleic acid expression regulatory sequence and a nucleotide sequence encoding a protein or RNA of interest are functionally linked to perform a general function. For example, a promoter and a nucleic acid sequence encoding a protein or RNA can be operably linked to affect expression of the coding sequence. A operative linkage with an expression vector can be produced using genetic recombination techniques well known in the art, and site-specific DNA cleavage and linkage can be carried out using enzymes and the like generally known in this art.

In addition, the expression vector may include a signal sequence for excretion of the peptide in order to facilitate the separation of the peptide from the cell culture solution. A specific initiation signal may also be required for efficient translation of an inserted nucleic acid sequence. These signals include an ATG initiation codon and adjacent sequences. In some cases, an exogenous translational control signal, which may include an ATG initiation codon, must be provided. These exogenous translation control signals and initiation codons can be from a variety of natural and synthetic sources. An expression efficiency can be increased by introduction of suitable transcriptional or translational enhancers.

In addition, the expression vector may further include a protein tag that can be optionally removed using endopeptidase to facilitate detection of the peptide.

The term "tag" as used herein refers to a molecule exhibiting a quantifiable activity or characteristic, is a chemical fluorescent substance (fluoracer) such as fluorescein, and polypeptide fluorophore such as fluorescent protein (GFP) or related proteins; and may be an epitope tag such as a Myc tag, a Flag tag, a histidine tag, a leucine tag, an IgG tag, or a streptavidin tag. In particular, when the epitope tag is used, peptide tags composed of preferably 6 or more amino acid residues, more preferably 8 to 50 amino acid residues may be used.

In the present invention, the expression vector includes a nucleotide sequence encoding the above-described peptide for promoting regeneration of of a hard tissue including dentin, bone and cementum and treating a dentin-dental pulp disease and/or a periodontal disease of the present invention, and the vector used at this time is not particularly limited thereto, as long as it can produce the peptide, and
more preferably, the vector may include commercially developed plasmids (pUC18, pBAD, pIDTSAMRT-AMP, etc.), Escherichia coli-derived plasmids (pYG601BR322, pBR325, pUC118, pUC119, etc.), Bacillus subtilis-derived plasmids (pUB110, pTP5, etc.), yeast-derived Plasmids (YEp13, YEp24, YCp50, etc.), phage DNA (Charon4A, Charon21A, EMBL3, EMBL4, λgt10, λgt11, λZAP, etc.), animal virus vectors (retrovirus, adenovirus, vaccinia virus), insect virus vectors (baculovirus, and the like). Since the expression vector shows different protein expression levels and modifications depending on the host cell, it is preferable to select and use a host cell most suitable for the purpose.

The transformant provided in the present invention can be prepared by introducing and transforming the expression vector provided in the present invention into the host, and can be used to produce the peptide by expressing the polynucleotide included in the expression vector. The transformation can be performed by various methods, but is not particularly limited thereto as long as the peptide can be produced, CaCl₂ precipitation, a Hanahan method with increased efficiency by using a reducing material called dimethyl sulfoxide (DMSO) in the CaCl₂ precipitation, electroporation, calcium phosphate precipitation, protoplast fusion, agitation using silicon carbide fibers, agrobacteria-mediated transformation, transformation using PED, dextran sulfate, lipofectamine and drying/suppression-mediated transformation and the like can be used. In addition, as long as the host used for preparing the transformant can also produce the peptide, it is not particularly limited thereto, but bacterial cells such as Escherichia coli, Streptomyces, and Salmonella typhimurium; yeast cells such as Saccharomyces cerevisiae and Schizosaccharomyces pombe; fungal cells such as Pichia pastoris; insect cells such as Drosophila and Spodoptera Sf9 cells; animal cells such as CHO, COS, NSO, 293, and Bow melanoma cells; or plant cells may be included.

The transformant may also be used in the method of producing the peptide for promoting regeneration of the hard tissue including dentin, bone and cementum and/or the dental pulp tissue and for treating the dentin-dental pulp disease and/or the periodontal disease. Specifically, the method of producing the peptide for promoting regeneration of the hard tissue including dentin, bone and cementum and/or the dental pulp tissue and for treating the dentin-dental pulp disease and/or the periodontal disease may include (a) culturing the transformant to obtain a culture; and (b) recovering the peptide of the present invention from the culture.

The term "culturing" of the present invention refers to a method of growing microorganisms under appropriately artificially controlled environmental conditions. In the present invention, the method for culturing the transformant may be performed using a method widely known in the art. Specifically, the culture is not particularly limited thereto as long as it can be produced by expressing the peptide for promoting regeneration of of the hard tissue including dentin, bone and cementum and treating the dentin-dental pulp disease and/or the periodontal disease of the present invention, and transformant may be cultured continuously in a batch process or fed batch or repeated fed batch process.

The medium used for culture must meet the requirements of a specific strain in an appropriate manner while controlling temperature, pH, and the like under aerobic conditions in a conventional medium including appropriate carbon sources, nitrogen sources, amino acids, vitamins, and the like. As a carbon source that can be used, a mixed sugar of glucose and xylose is used as a main carbon source, including sugars and carbohydrates such as sucrose, lactose, fructose, maltose, starch, and cellulose, oils and fats such as soybean oil, sunflower oil, castor oil, and coconut oil, fatty acids such as palmitic acid, stearic acid, linoleic acid, and the like, alcohols such as glycerol and ethanol, and organic acids such as acetic acids. These materials may be used individually or as a mixture. Nitrogen sources that can be used may include inorganic nitrogen sources such as ammonia, ammonium sulfate, ammonium chloride, ammonium acetate, ammonium phosphate, ammonium carbonate, and ammonium nitrate; amino acids such as glutamic acid, methionine, and glutamine, and organic nitrogen sources such as peptone, NZ-amine, meat extract, yeast extract, malt extract, corn steep liquor, casein hydrolysate, fish or decomposition products thereof, defatted soybean cake, or decomposition products thereof. These nitrogen sources may be used alone or in combination. The medium may include monopotassium phosphate, dipotassium phosphate and the corresponding sodium-containing salts as a phosphorus source. Persons that may be used include potassium dihydrogen phosphate or dipotassium hydrogen phosphate or the corresponding sodium-containing salts. In addition, as the inorganic compound, sodium chloride, calcium chloride, iron chloride, magnesium sulfate, iron sulfate, manganese sulfate, and calcium carbonate may be used. Finally, essential growth substances such as amino acids and vitamins may be used in addition to the above substances.

In addition, precursors suitable for the culture medium may be used. The above raw materials may be added in a batch, fed-batch or continuous manner by a method suitable for the culture during a culture process, but is not particularly limited thereto. Basic compounds such as sodium hydroxide, potassium hydroxide, ammonia or acid compounds such as phosphoric acid or sulfuric acid can be used in an appropriate manner to adjust a pH of the culture.

In addition, antifoaming agents such as fatty acid polyglycol esters may be used to suppress foam formation. Oxygen or an oxygen-containing gas (for example, air) is injected into the culture to maintain aerobic conditions. A temperature of the culture is usually 27°C to 37°C, preferably 30°C to 35°C. The culture is continued until the maximum production of the peptide is obtained. For this purpose, it is usually achieved in 10 to 100 hours.

In addition, recovering the peptide from the culture may be performed by a method known in the art. Specifically, the recovery method is not particularly limited thereto as long as it can be used for recovery of the produced peptide, but preferably centrifugation, filtration, extraction, spraying, drying, steaming, precipitation, crystallization, electrophoresis, fractional dissolution methods such as (for example, ammonium sulfate precipitation), chromatography (for example, ion exchange, affinity, hydrophobicity and size exclusion) may be used.

As another aspect, the present invention provides a pharmaceutical composition for preventing or treating a dentin-dental pulp disease, including the peptide.

As described above, when the peptide for promoting regeneration of the hard tissue including dentin, bone and cementum and/or the dental pulp tissue, and treating the dentin-dental pulp disease and/or the periodontal disease of the present invention is transplanted in vivo together with human dental pulp cells, since the human dental pulp cells can promote the formation of a dentin/dental pulp tissue-like tissue, it can be used as an active ingredient of a pharmaceutical composition for treating the dentin-dental pulp disease caused by damage to the pulp tissue.

The peptide included in the pharmaceutical composition may be used in the form of a peptide alone, or may be used in the form of a polypeptide in which the peptide is repeated two or more times, and it may also be used in the form of a complex in which a drug exhibiting a therapeutic effect for the dentin-dental pulp disease is bound to the N-terminus or C-terminus of the peptide.

The term "dentin-dental pulp disease" of the present invention refers to a disease caused by damage to the pulp tissue and dentin coupled thereto due to damage to the pulp tissue.

**In** the present invention, the dentin-dental pulp disease is not particularly limited thereto as long as the peptide of the present invention exhibits a therapeutic effect, but as an example, dentine hyperesthesia, pulp hyperemia, pulpitis, pulp modification, pulp necrosis and gangrene, and the like.

As another aspect, the present invention provides a pharmaceutical composition for preventing or treating periodontal disease, including the peptide.

As described above, when the peptide for promoting regeneration of the hard tissue including dentin, bone and cementum and/or the dental pulp tissue, and treating the dentin-dental pulp disease and/or the periodontal disease of the present invention is transplanted in vivo together with human dental pulp cells, since the human dental pulp cells can promote the formation of a bone-like tissue, it can be used as an active ingredient of a pharmaceutical composition for treating the periodontal disease that causes bone and/or cementum damage.

The peptide included in the pharmaceutical composition may be used in the form of a peptide alone, or may be used in the form of a polypeptide in which the peptide is repeated two or more times, and it may also be used in the form of a complex in which a drug exhibiting a therapeutic effect for the periodontal disease is bound to the N-terminus or C-terminus of the peptide.

The term "periodontal disease" of the present invention, also called a gum disease, refers to a disease in which bacteria infect the gap between gingiva (gum) and teeth and damage the periodontal ligament and adjacent tissues, and it is divided into gingivitis and periodontitis. At the onset of the periodontal disease, inflammation progresses and more tissues are damaged, forming a periodontal pocket, and the deeper the periodontitis, the deeper the periodontal pocket, and it is known that as the periodontal pocket deepens, the periodontal ligament becomes inflamed and finally bone loss is induced.

In the present invention, the periodontal disease is not particularly limited as long as the peptide of the present invention exhibits a therapeutic effect, but as an example, gingivitis, periodontitis, periodontal pocket or periodontal abscess abscess) may be included.

The term "prevention" of the present invention refers to any action that inhibits or delays the occurrence of the dentin-dental pulp disease by administering the pharmaceutical composition for preventing or treating a dentin-pulp tissue disease, including the peptide of the present invention, or any action that inhibits or delays the occurrence of the periodontal disease by administering the pharmaceutical composition for preventing or treating the periodontal disease, including the peptide of the present invention.

The term "treatment" of the present invention refers to any action that promote the regeneration of a dentin or dental pulp tissue to treat the pulp disease by administering a pharmaceutical composition including the peptide of the present invention as an active ingredient to a subject in need of treatment for the dentin-dental pulp disease, or any action that promotes regeneration of bone and/or cementum to treat the periodontal disease by administering a pharmaceutical composition including the peptide of the present invention as an active ingredient to a subject in need of treatment for the periodontal disease.

The pharmaceutical composition of the present invention may be prepared in the form of a pharmaceutical composition for treating the dentin-dental pulp disease and/or the periodontal disease, further including an appropriate carrier (natural or non-natural carrier), excipient or diluent commonly used in the preparation of pharmaceutical compositions to the peptide. Specifically, the pharmaceutical composition can be formulated and used in the form of a sterile injectable solution, each of which can be administered to fatty liver disease and/or a site induced by the dentin-dental pulp disease and/or the periodontal disease to conventional methods. **In** the present invention, carriers, excipients and diluents that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, collagen, etc. **In** the case of formulation, it can be prepared using diluents or excipients such as fillers, extenders, binders, wetting agents, disintegrating agents and surfactants usually used. In particular, sterile aqueous solutions, non-aqueous solvents, suspensions, emulsions, lyophilized preparations, suppositories, ointments (e.g., dimensional liner, etc.), etc. may be included. As the non-aqueous solvent and suspending agent, propylene glycol, polyethylene glycol, vegetable oils such as olive oil, injectable esters such as ethyl oleate, etc. can be used. As the suppository base, witepsol, Macrogol, tween 61, cacao butter, laurin butter, glycerogeratin, etc. can be used.

A content of the peptide included in the pharmaceutical composition of the present invention is not particularly limited, but may be contained in an amount of 0.0001 to 50 wt %, more preferably 0.01 to 20 wt %, based on the total weight of the final composition.

The pharmaceutical composition of the present invention can be administered in a pharmaceutically effective amount, and the term "pharmaceutically effective amount" means an amount sufficient to treat or prevent a disease at a reasonable benefit/risk ratio applicable to medical treatment or prevention, and an effective dose level can be determined according to a severity of a disease, an activity of a drug, age, body weight, health, sex of a patient, sensitivity to patient's drugs, time of administration, route of administration and a duration of a treatment of a rate of excretion of the inventive composition employed, factors including the drugs used in combination or coincidental with the inventive compositions employed, and other factors well known in the medical arts. The pharmaceutical composition of the present invention may be administered alone or in combination the pharmaceutical composition for treating the known dentin-dental pulp disease and/or the periodontal disease. Considering all of the above factors, it is important to administer a minimum amount of the maximum effect without side effects.

The dosage of the pharmaceutical composition of the present invention can be determined by those skilled in the art in consideration of the purpose of use, the severity of the disease, the age, body weight, sex, history of the patient, or the kind of the substance used as an active component. For example, the pharmaceutical composition of the present invention can be administered at about 0.1 ng to about 100 mg/kg, preferably 1 ng to 10 mg/kg, per adult, and the dosage frequency of the composition is not particularly limited, but may be administered once a day or several divided doses. The above dosages do not limit the scope of the invention in any respect.

As another aspect, the present invention provides a method of treating the dentin-dental pulp disease, including administering a pharmaceutically effective amount of the pharmaceutical composition to a non-human subject having the dentin-dental pulp disease. In another aspect, there is provided a method of treating the periodontal disease, including administering a pharmaceutically effective amount of the pharmaceutical composition to a non-human subject having the periodontal disease.

The term "individual" of the present invention may include, without limitation, a human in need of treatment for a dentin-dental pulp disease and/or a periodontal disease, or a mammal, including non-human rats, livestock, etc.

The route of administration of the pharmaceutical composition for treating the dentin-dental pulp disease and/or the periodontal disease of the present invention may be administered via any general route as long as the desired tissue can be reached. The pharmaceutical composition of the present invention is not particularly limited, but may be administered as desired via a route such as oral administration or oral injection.

As another aspect, the present invention provides a quasi-drug composition for preventing or improving the periodontal disease, including the peptide, or an quasi-drug composition for preventing or improving fatty liver, including the peptide.

As used herein, the term "improvement" refers to any act of reducing at least the extent of a parameter, e.g., a symptom, associated with a condition being treated.

In the present invention, the improvement refers to any action that promote the regeneration of a dentin or dental pulp tissue to ameliorate or benefit the symptoms of the dentin-dental pulp disease by administering a pharmaceutical composition including the peptide of the present invention as an active ingredient to a subject in need of treatment for the dentin-dental pulp disease, or any action that promotes regeneration of bone and/or cementum to ameliorate or benefit the symptoms of the periodontal disease by administering a pharmaceutical composition including the peptide of the present invention as an active ingredient to a subject in need of treatment for the periodontal disease.

The term "quasi-drug" of the present invention refers to articles that are milder in action than pharmaceuticals, among articles used for the purpose of diagnosing, treating, ameliorating, alleviating, treating or preventing a disease in a human or animal, for example, according to the pharmacist method, the quasi-drug is a substance other than an article used for a pharmaceutical, and includes a fiber/rubber product used for treating or preventing diseases of humans/animals, a substance similar to a substance that has little or no direct action on the human body and is not a mechanism or a machine, and a bactericidal/pesticidal agent for preventing infectious diseases.

In the present invedntion, the type or formulation of the quasi-drug composition including the peptide is not particularly limited, but as an example, it may be an oral disinfectant, an oral cleaning product, a toothpaste, a dental floss, an oral ointment, and the like.

As another aspect, the present invention provides a health functional food composition for preventing or improving the dentin-dental pulp disease and/or the periodontal disease, including the peptide.

The term "food" of the present invention includes dairy products including meat, sausage, bread, chocolate, candys, snacks, confectionary, pizza, ramie, other noodles, gums, ice cream, various soups, beverages, teas, drinks, alcohol drinks, vitamin complexes, health functional foods and health foods, etc., and includes all foods in a conventional sense.

The above health functional food is the same term as the food for special health use (FoSHU), and means a food product having a high medical and medical effect processed so that bioregulatory functions are efficiently exhibited in addition to nutrient supply. The term "function(ality)" as used herein means to control nutrients for the structure and function of the human body or to obtain a useful effect for health uses such as physiological action. The food of the present invention can be produced by a method commonly used in the art, and in such production, raw materials and components commonly added in the industry can be added. The formulation of the food can also be prepared without limitation, provided that it is a formulation recognized as a food. The composition for food of the present invention can be prepared in various forms of dosage form, has the advantage of using food as a raw material unlike general drugs and having no side effects, etc. that can occur during long-term dosing of drugs, and is excellent in carrying property, and the food of this invention can also be taken as an adjuvant for enhancing the effect of preventing or improving the dentin-dental pulp disease and/or the periodontal disease.

The term "health functional food" refers to a food product having an active health maintenance and an enhancing effect as compared with the general formula product, and a health supplement food refers to a food product for a health supplement purpose. In some cases, the terms health functional food, health food and health auxiliary food may be used interchangeably.

Specifically, the above health functional food means a food product prepared by adding a peptide to a food material such as a beverage, a tea, a flavoring agent, a gum, and a confectionery, or by encapsulating, pulverizing, suspending or the like, which brings about a certain effect on health when ingested, but it has an advantage that it does not have any adverse effect that can occur when a food product is used as a raw material for a long-term administration of a drug, unlike general drugs.

Since the food composition of the present invention can be consumed on a daily basis, a high effect can be expected for the prevention or improvement of the dentin-dental pulp disease and/or the periodontal disease, and thus it can be very usefully used.

The food composition may further include a physiologically acceptable carrier, and the kind of the carrier is not particularly limited, and any carrier commonly used in the art may be used.

In addition, the food composition may include additional components that are commonly used in food compositions to enhance odor, taste, vision, etc. For example, vitamin A, C, D, E, B1, B2, B6, B12, niacin, biotin, folate, pantothenic acid, etc. may be included. It may also include minerals such as zinc (Zn), iron (Fe), calcium (Ca), chromium (Cr), magnesium (Mg), manganese (Mn), copper (Cu), and cerium (Cr). It may also contain amino acids such as lysine, tryptophan, cysteine, valine, etc.

In addition,the food composition may contain food additives such as antiseptics (potassium sorbate, sodium benzoate, salicylic acid, sodium dehydrocholate, etc.), bactericides (bleaching powder, high bleaching powder, sodium hypochlorite, etc.), antioxidants (butylhydroxyanisole (BHA), butylhydroxytolene (BHT), etc.) coloring agents (tar coloring agent, etc,), color-forming agents (sodium nitrite, sodium subacetate, etc). bleaching agent (sodium sulfite), seasonings (sodium MSG glutamate, etc), sweeteners (dulcine, cyclamate, saccharin, sodium, etc) perfumes (vanillin, lactones, etc); expanding agents (alum, potassium D-tartrate, etc); reinforcing agents, emulsifiers, thickeners (daments), coating agents, screening agents, foam inhibitors, solvents, and conditioners. The additives are selected according to the kind of food and can be used in an appropriate amount.

The peptide of the present invention can be added as it is or used in combination with other food or food components, and can be suitably used according to a conventional method. The mixed amount of the active component can be suitably determined according to the purpose of its use (prevention, health or therapeutic treatment). Generally, in the manufacture of a food or beverage, the food composition of the present invention may be added in an amount of 50 parts by weight or less, specifically 20 parts by weight or less, relative to the food or beverage. However, when ingested for a long period of time for the purpose of health and hygiene, the content may be within the above range, and since there is no problem in terms of safety, the active component may also be used in an amount above the above range.

One example of the food composition of the present invention may be used as a health beverage composition, in which case it may contain as additional components various flavoring agents, natural carbohydrates, etc., such as conventional beverages. The above-mentioned natural carbohydrates may be monosaccharides such as glucose, fructose; disaccharides such as maltose, sucrose; polysaccharides such as dextrin, cyclodextrin; sugar alcohols such as xylitol, sorbitol, erythritol, etc. As the sweetening agent, natural sweetening agents such as thaumatin, stevia extract; synthetic sweeteners such as saccharin, aspartame, etc. can be used. The proportion of natural carbohydrates can generally be about 0.01 to 0.04 g, specifically about 0.02 to 0.03 g, per 100 ml of the health drink composition of the present invention.

In addition to the above, the health drink composition may contain various nutrients, vitamins, electrolytes, flavoring agents, coloring agents, pectic acid, salts of pecting acid, alginic acid, salt of alginic acids, organic acids, protective colloid thickeners, pH regulators, stabilizers, antiseptics, glycerin, alcohols, carbonating agents, etc. They may also contain flesh for the production of natural fruit juices, fruit juice beverages, or vegetable beverages. These components may be used independently or in combination. The proportion of such additives is not critical but is generally selected in the range of 0.01 to 0.1 parts by weight per 100 parts by weight of the health and beverage composition of the invention.

The food composition of the invention may include various weight %s as long as the effect of preventing or ameliorating the dentin-dental pulp disease and/or the periodontal disease is exhibited, but specifically, it may include 0.00001 to 100 weight % or 0.01 to 80 weight % of the peptide of the present invention, relative to the total weight of the food composition, but is not limited thereto.

In another aspect of the present invention, there is provided a method for preventing or treating the dentin-dental pulp disease, and/or the periodontal disease, including administering to the subject a composition, including the peptide.

As another aspect, there is provided a method for promoting regeneration of a dentin or dental pulp tissue, and/or a method for promoting regeneration of bone or cementum, including administering a composition including the peptide to a subject.

As another aspect of the present invention, there is provided use for promoting regeneration of of a hard tissue including dentin, bone and cementum and/or a dental pulp tissue of a peptide including an amino acid sequence of the General formula 1 below or a composition comprising the peptide, and use for preventing or treating the dentin-dental pulp disease or the periodontal disease.

K-Y-R1-R2-R3-R4-R5-R6-Y-K (General formula 1)

In General formula 1, R1 and R2 are each lysine (K), alanine (A) or arginine (R), R3, R4, and R5 are each lysine (K) or arginine (R), and R6 is asparagine (N ) or serine (S).

As another aspect of the present invention, there is provided use for promoting regeneration of of a hard tissue including dentin, bone and cementum and/or a dental pulp tissue of a peptide including an amino acid sequence of any one of SEQ ID NOs: 1 to 64 or a composition comprising the peptide, and/or use for preventing or treating the dentin-dental pulp disease or the periodontal disease.

In addition, as one embodiment of the present invention, there is provided use for promoting regeneration of of a hard tissue including dentin, bone and cementum and/or a dental pulp tissue of a peptide including an amino acid sequence of any one of SEQ ID NOs: 1 to 8 or a composition including the peptide, and/or use for preventing or treating the dentin-dental pulp disease or the periodontal disease.

Hereinafter, the present invention will be described in detail by way of Examples. However, these Examples are for illustrative purposes of the present invention, and the scope of the present invention is not limited to these Examples.

### Example 1: Experimental method and material

### Example 1-1. Synthesis of peptides for promoting regeneration of hard tissue including dentin, bone and cementum and/or dental pulp tissue and treating the dentin-dental pulp disease and/or the periodontal disease

The present inventors synthesized a peptide (SEQ ID NO: 1) exhibiting an effect of promoting regeneration of a hard tissue including dentin, bone and cementum and/or a dental pulp tissue using the 9-fluorenylmethyloxycarbonyl (Fmoc) method, and the peptide of each group were synthesized by substituting an amino acid of the synthesized peptide (Tables 1 to 8).
N-KYKAKKKNYK-C (SEQ ID NO: 1)

First, peptides of group 1 were synthesized by substituting the peptide of SEQ ID NO: 1 or amino acids 5 to 7 of the peptide of SEQ ID NO: 1 with arginine (Table 1).

**Table 1. Peptides of group 1**

| SEQ ID NO | Amino acid sequence (N-C) |
|---|---|
| 1 | KYKAKKKNYK |
| 2 | KYKAKKRNYK |
| 3 | KYKAKRKNYK |
| 4 | KYKARKKNYK |
| 5 | KYKAKRRNYK |
| 6 | KYKARRKNYK |
| 7 | KYKARRRNYK |
| 8 | KYKARKRNYK |

Next, the peptides of group 1 were synthesized by substituting amino acid 8 of the peptide of SEQ ID NO: 1 with serine, and amino acids 5 to 7 with arginine (Table 2).

**Table 2. Peptides of group 2**

| SEQ ID NO | Amino acid sequence (N-C) |
|---|---|
| 9 | KYKAKKKSYK |
| 10 | KYKAKKRSYK |
| 11 | KYKAKRKSYK |
| 12 | KYKARKKSYK |
| 13 | KYKAKRRSYK |
| 14 | KYKARRKSYK |
| 15 | KYKARRRSYK |
| 16 | KYKARKRSYK |

The peptides of group 3 were synthesized by substituting amino acid 3 of the peptide of SEQ ID NO: 1 with arginine, and amino acids 5 to 7 with arginine (Table 3).

**Table 3. Peptides of group 3**

| SEQ ID NO | Amino acid sequence (N-C) |
|---|---|
| 17 | KYRAKKKNYK |
| 18 | KYRAKKRNYK |
| 19 | KYRAKRKNYK |
| 20 | KYRARKKNYK |
| 21 | KYRAKRRNYK |
| 22 | KYRARRKNYK |
| 23 | KYRARRRNYK |
| 24 | KYRARKRNYK |

The peptides of group 4 were synthesized by substituting amino acid 3 of the peptide of SEQ ID NO: 1 with arginine, amino acid 8 with serine, and amino acids 5 to 7 with arginine (Table 4).

**Table 4. Peptides of group 4**

| SEQ ID NO | Amino acid sequence (N-C) |
|---|---|
| 25 | KYRAKKKSYK |
| 26 | KYRAKKRSYK |
| 27 | KYRAKRKSYK |
| 28 | KYRARKKSYK |
| 29 | KYRAKRRSYK |
| 30 | KYRARRKSYK |
| 31 | KYRARRRSYK |
| 32 | KYRARKRSYK |

The peptides of group 5 were synthesized by substituting amino acid 3 of the peptide of SEQ ID NO: 1 with alanine, amino acid 4 with lysine, and amino acids 5 to 7 with arginine (Table 5).

**Table 5. Peptides of group 5**

| SEQ ID NO | Amino acid sequence (N-C) |
|---|---|
| 33 | KYAKKKKNYK |
| 34 | KYAKKKRNYK |
| 35 | KYAKKRKNYK |
| 36 | KYAKRKKNYK |
| 37 | KYAKKRRNYK |
| 38 | KYAKRRKNYK |
| 39 | KYAKRRRNYK |
| 40 | KYAKRKRNYK |

The peptides of group 6 were synthesized by substituting amino acid 3 of the peptide of SEQ ID NO: 1 with alanine, amino acid 4 with lysine, amino acid 8 with serine, and amino acids 5 to 7 with arginine (Table 6).

**Table 6. Peptides of group 6**

| SEQ ID NO | Amino acid sequence (N-C) |
|---|---|
| 41 | KYAKKKKSYK |
| 42 | KYAKKKRSYK |
| 43 | KYAKKRKSYK |
| 44 | KYAKRKKSYK |
| 45 | KYAKKRRSYK |
| 46 | KYAKRRKSYK |
| 47 | KYAKRRRSYK |
| 48 | KYAKRKRSYK |

The peptides of group 7 were synthesized by substituting amino acid 3 of the peptide of SEQ ID NO: 1 with alanine, amino acid 4 with arginine, and amino acids 5 to 7 with arginine (Table 7).

**Table 7. Peptides of group 7**

| SEQ ID NO | Amino acid sequence (N-C) |
|---|---|
| 49 | KYARKKKNYK |
| 50 | KYARKKRNYK |
| 51 | KYARKRKNYK |
| 52 | KYARRKKNYK |
| 53 | KYARKRRNYK |
| 54 | KYARRRKNYK |
| 55 | KYARRRRNYK |
| 56 | KYARRKRNYK |

The peptides of group 8 were synthesized by substituting amino acid 3 of the peptide of SEQ ID NO: 1 with alanine, amino acid 4 with arginine, amino acid 8 with serine, and amino acids 5 to 7 with arginine (Table 8).

**Table 8. Peptides of group 8**

| SEQ ID NO | Amino acid sequence (N-C) |
|---|---|
| 57 | KYARKKKSYK |
| 58 | KYARKKRSYK |
| 59 | KYARKRKSYK |
| 60 | KYARRKKSYK |
| 61 | KYARKRRSYK |
| 62 | KYARRRKSYK |
| 63 | KYARRRRSYK |
| 64 | KYARRKRSYK |

### Example 1-2. Cell culture

It was cultured at 37°C in humid air containing 5% CO₂ and used for the experiment. Human mesenchymal stem cells (hBMSCs) were purchased from Lonza (LONZA, Switzerland) and used. hBMSCs were cultured in α-MEM (Invitrogen) medium supplemented with 10% heatinactivated bovine serum.

### Example 1-3. Isolation and culture of human-derived dental pulp cells

Human pulp tissue cells were isolated from wisdom teeth of 10 adults (18-22 years old) at Seoul National University Dental Hospital. Specifically, all experiments were performed with the patient's consent after approval from the hospital's Institutional Review Board, and wisdom teeth are cut according to a method of Jung HS et al (J Mol Histol. (2011)), exposing the pulp and isolating the pulp with forceps. The isolated pulp was minced on both sides, placed in a 60 mm dish, covered with a cover slip, and then cultured in Dulbecco's Modified Eagle medium. It is known that human dental pulp cells can differentiate into odontoblasts, osteoblasts, cementoblasts and periodontal ligament cells under various conditions (Tissue Eng Part A. 2014 Apr;20(7-8):1342-51).

### Example 1-4. Reverse transcription-polymerase chain reaction (RT-PCR) and real-time PCR analysis

Total RNA of human dental pulp cells and mesenchymal stem cells was isolated using a TRIzol reagent. cDNA was synthesized using 2 µg of total RNA, 1 µl of reverse transcriptase, and 0.5 µg of oligo (dT). The synthesized cDNA was used for real-time polymerase chain reaction. The real-time polymerase chain reaction was performed in ABI PRISM 7500 sequence detection system (Applied Biosystems) using SYBR GREEN PCR Master Mix (Takara, Japan). The real-time polymerase chain reaction was performed at 94 °C, 1 min; 95 °C, 15 sec - 60 °C, 34 sec was performed under the condition of repeating 40 cycles. An analysis of the results was evaluated using a comparative cycle threshold (CT) method, and primer base sequences are listed in Table 9.

**Table 9. Nucleotide Sequences of Real-Time PCR Primers**

| Gene | Primer (5'-3') | |
|---|---|---|
| *hBSP* | forward | GAATGGCCTGTGCTTTCTCAA |
| | reverse | TCGGATGAGTCACTACTGCCC |
| *hDMP1* | forward | ACAGGCAAATGAAGACCC |
| | reverse | TTCACTGGCTTGTATGG |
| *hCAP* | forward | GACGAGGACGGCACCAACGG |
| | reverse | CGCGGTCATGGCGATGTCGT |
| *hDSPP* | forward | CAACCATAGAGAAAGCAAAC GCG |
| | reverse | TTTCTGTTGCCACTGCTGGGA C |
| hGAPD H | forward | CCATGGAGAAGGCTGGGG |
| | reverse | CAAAGTTCTCATGGATGACC |

### Example 1-5. In vivo transplantation and histological analysis

Human dental pulp cells (hDPCs) were isolated and used for in vivo transplantation experiments. hDPCs (2 x 10⁶ cells) were mixed with 100 mg of hydroxy apatite/tricalcium phosphate (HA/TCP) ceramic powder (Zimmer, USA) alone or along with peptides (10 µg) or 0.5% fibrin gel, and then transplanted into mice (NIH-bg-nu-xid; Harlan Laboratories, Indianapolis, IN) with damaged immune systems for 6 and 12 weeks. Thereafter, samples were harvested, fixed in 4% paraformaldehyde, decalcified in 10% EDTA (pH 7.4), embedded in paraffin, and stained with hematoxylin-eosin (H-E) (Vector Labs).

### Example 1-6. Effect of novel peptide on dentin regeneration in dentin damage model

Three beagle dogs (12-16 kg; 6-8 weeks old) were anesthetized by inhalation of Geloran, intravenously injected with Zoletil (5 mg/kg) and Xylazine (0.2-0.5 mg/kg), and then treated with Lidocaine (Lidocaine 2% with 1:80,000 epinephrine). After damaging the dentin of the premolars and molar teeth in the mandible of the beagle dog using a dental bur, 10 ug of SEQ ID NO: 1 peptide of group 1 was treated per tooth. After 6 weeks, a beagle dog was sacrificed by administering an excessive amount (90-120 mg/kg) of pentobarbital. The tooth of the beagle dog was extracted, fixed with 10% formalin, calcium was removed by adding 5% formic acid, molded, and embedded in paraffin, and then a tissue section with a thickness of 5 µm were obtained, a obtained tissue section was stained with hematoxylin and eosin, and then analyzed using by an optical microscope equipped with a digital camera (LEICA ICC50 camera, Germany).

A statistical analysis in the examples of the present invention was performed using Student's t-test, and SPSS software ver. 19.0 was used.

### - Effect of peptide for promoting regeneration of dentin or dental pulp tissue and treating dentine hyperesthesia on an expression level of DSPP gene which is a marker gene for odontoblast differentiation

The DSPP gene is used as an odontoblast differentiation marker and is known to be an important gene for dentin calcification. Therefore, in this study, a novel peptide that promotes odontoblast differentiation and dentin formation by increasing the expression of the DSPP gene which is the marker gene for odontoblast differentiation, was synthesized and the effect was confirmed.

The effect of each group of peptides on DSPP mRNA expression was confirmed by real-time PCR (Table 10).

**Table 10. Effect of peptides of groups 1 to 9 on mRNA level of DSPP gene**

| BSP gene expression | | | |
|---|---|---|---|
| | Sequence (5'-3') | SEQ ID NO | Average |
| Group 1 | KYKAKKKNYK | 1 | 9.028 |
| | KYKAKKRNYK | 2 | 7.361 |
| | KYKAKRKNYK | 3 | 8.572 |
| | KYKARKKNYK | 4 | 9.702 |
| | KYKAKRRNYK | 5 | 6.67 |
| | KYKARRKNYK | 6 | 7.705 |
| | KYKARRRNYK | 7 | 8.215 |
| | KYKARKRNYK | 8 | 7.021 |
| Group 2 | KYKAKKKSYK | 9 | 3.057 |
| | KYKAKKRSYK | 10 | 3.811 |
| | KYKAKRKSYK | 11 | 3.362 |
| | KYKARKKSYK | 12 | 2.911 |
| | KYKAKRRSYK | 13 | 2.535 |
| | KYKARRKSYK | 14 | 2.136 |
| | KYKARRRSYK | 15 | 3.620 |
| | KYKARKRSYK | 16 | 2.906 |
| Group 3 | KYRAKKKNYK | 17 | 2.192 |
| | KYRAKKRNYK | 18 | 2.061 |
| | KYRAKRKNYK | 19 | 1.772 |
| | KYRARKKNYK | 20 | 1.702 |
| | KYRAKRRNYK | 21 | 2.167 |
| | KYRARRKNYK | 22 | 2.005 |
| | KYRARRRNYK | 23 | 1.915 |
| | KYRARKRNYK | 24 | 2.021 |
| Group 4 | KYRAKKKSYK | 25 | 1.919 |
| | KYRAKKRSYK | 26 | 1.811 |
| | KYRAKRKSYK | 27 | 1.620 |
| | KYRARKKSYK | 28 | 2.311 |
| | KYRAKRRSYK | 29 | 2.125 |
| | KYRARRKSYK | 30 | 1.836 |
| | KYRARRRSYK | 31 | 1.620 |
| | KYRARKRSYK | 32 | 1.861 |
| Group 5 | KYAKKKKNYK | 33 | 1.813 |
| | KYAKKKRNYK | 34 | 2.361 |
| | KYAKKRKNYK | 35 | 1.572 |
| | KYAKRKKNYK | 36 | 1.702 |
| | KYAKKRRNYK | 37 | 1.67 |
| | KYAKRRKNYK | 38 | 1.705 |
| | KYAKRRRNYK | 39 | 2.015 |
| | KYAKRKRNYK | 40 | 2.121 |
| Group 6 | KYAKKKKSYK | 41 | 1.897 |
| | KYAKKKRSYK | 42 | 1.811 |
| | KYAKKRKSYK | 43 | 1.620 |
| | KYAKRKKSYK | 44 | 2.121 |
| | KYAKKRRSYK | 45 | 2.125 |
| | KYAKRRKSYK | 46 | 1.836 |
| | KYAKRRRSYK | 47 | 1.620 |
| | KYAKRKRSYK | 48 | 2.516 |
| Group 7 | KYARKKKNYK | 49 | 1.798 |
| | KYARKKRNYK | 50 | 1.661 |
| | KYARKRKNYK | 51 | 2.572 |
| | KYARRKKNYK | 52 | 2.102 |
| | KYARKRRNYK | 53 | 2.037 |
| | KYARRRKNYK | 54 | 2.005 |
| | KYARRRRNYK | 55 | 1.921 |
| | KYARRKRNYK | 56 | 2.021 |
| Group 8 | KYARKKKSYK | 57 | 1.886 |
| | KYARKKRSYK | 58 | 1.811 |
| | KYARKRKSYK | 59 | 2.120 |
| | KYARRKKSYK | 60 | 2.195 |
| | KYARKRRSYK | 61 | 1.525 |
| | KYARRRKSYK | 62 | 1.836 |
| | KYARRRRSYK | 63 | 2.120 |
| | KYARRKRSYK | 64 | 1.606 |

FIG. 1 is a graph showing the average of DSPP mRNA expression values in each group of Table 10. In FIG. 1, DSPP mRNA expression was increased by about 2 to 8 times in all peptide groups compared to a control group, and in particular, a peptide group in group 1 showed the highest DSPP mRNA expression value. Since each of differentiation marker genes is known to be a gene involved in odontoblast differentiation and dentin calcification, it was analyzed that the peptide provided in the present invention has an effect of promoting dentin regeneration.

### - Effect of peptides for promoting bone or cementum regeneration and treating periodontal disease on the expression level of BSP gene which is a marker gene for osteoblast and cementoblast differentiation

BSP and DMP1 genes are used as markers for differentiation of osteoblasts and cementoblasts, and are known as important genes for calcification of a bone and cementum. The effects of each group of peptides on BSP and DMP1 mRNA expression were confirmed by a real-time PCR (Tables 11 and 12).

**Table 11. Effect of peptides of groups 1 to 8 on mRNA level of BSP gene**

| BSP gene expression | | | |
|---|---|---|---|
| | Sequence (5'-3') | SEQ ID NO | Average |
| Group 1 | KYKAKKKNYK | 1 | 2.892 |
| | KYKAKKRNYK | 2 | 2.713 |
| | KYKAKRKNYK | 3 | 2.715 |
| | KYKARKKNYK | 4 | 2.307 |
| | KYKAKRRNYK | 5 | 2.77 |
| | KYKARRKNYK | 6 | 2.935 |
| | KYKARRRNYK | 7 | 2.548 |
| | KYKARKRNYK | 8 | 2.617 |
| Group 2 | KYKAKKKSYK | 9 | 2.084 |
| | KYKAKKRSYK | 10 | 1.998 |
| | KYKAKRKSYK | 11 | 2.226 |
| | KYKARKKSYK | 12 | 1.918 |
| | KYKAKRRSYK | 13 | 2.637 |
| | KYKARRKSYK | 14 | 1.729 |
| | KYKARRRSYK | 15 | 2.119 |
| | KYKARKRSYK | 16 | 2.307 |
| Group 3 | KYRAKKKNYK | 17 | 2.392 |
| | KYRAKKRNYK | 18 | 2.316 |
| | KYRAKRKNYK | 19 | 2.537 |
| | KYRARKKNYK | 20 | 2.91 |
| | KYRAKRRNYK | 21 | 1.995 |
| | KYRARRKNYK | 22 | 1.893 |
| | KYRARRRNYK | 23 | 2.037 |
| | KYRARKRNYK | 24 | 2.276 |
| Group 4 | KYRAKKKSYK | 25 | 2.152 |
| | KYRAKKRSYK | 26 | 2.081 |
| | KYRAKRKSYK | 27 | 2.195 |
| | KYRARKKSYK | 28 | 2.084 |
| | KYRAKRRSYK | 29 | 1.978 |
| | KYRARRKSYK | 30 | 2.222 |
| | KYRARRRSYK | 31 | 1.798 |
| | KYRARKRSYK | 32 | 1.893 |
| Group 5 | KYAKKKKNYK | 33 | 2.092 |
| | KYAKKKRNYK | 34 | 2.136 |
| | KYAKKRKNYK | 35 | 2.372 |
| | KYAKRKKNYK | 36 | 2.207 |
| | KYAKKRRNYK | 37 | 1.999 |
| | KYAKRRKNYK | 38 | 2.507 |
| | KYAKRRRNYK | 39 | 2.293 |
| | KYAKRKRNYK | 40 | 2.102 |
| Group 6 | KYAKKKKSYK | 41 | 1.762 |
| | KYAKKKRSYK | 42 | 1.882 |
| | KYAKKRKSYK | 43 | 2.011 |
| | KYAKRKKSYK | 44 | 1.699 |
| | KYAKKRRSYK | 45 | 1.878 |
| | KYAKRRKSYK | 46 | 1.863 |
| | KYAKRRRSYK | 47 | 2.063 |
| | KYAKRKRSYK | 48 | 1.773 |
| Group 7 | KYARKKKNYK | 49 | 1.971 |
| | KYARKKRNYK | 50 | 1.901 |
| | KYARKRKNYK | 51 | 1.899 |
| | KYARRKKNYK | 52 | 1.999 |
| | KYARKRRNYK | 53 | 2.036 |
| | KYARRRKNYK | 54 | 2.1 |
| | KYARRRRNYK | 55 | 1.773 |
| | KYARRKRNYK | 56 | 2.016 |
| Group 8 | KYARKKKSYK | 57 | 1.679 |
| | KYARKKRSYK | 58 | 1.811 |
| | KYARKRKSYK | 59 | 1.629 |
| | KYARRKKSYK | 60 | 1.991 |
| | KYARKRRSYK | 61 | 1.832 |
| | KYARRRKSYK | 62 | 1.769 |
| | KYARRRRSYK | 63 | 2.237 |
| | KYARRKRSYK | 64 | 2.021 |

**Table 12. Effect of peptides of groups 1 to 9 on mRNA level of DMP1 gene**

| DMP1 gene expression | | | |
|---|---|---|---|
| | Sequence | SEQ ID NO | Average |
| Group 1 | KYKAKKKNYK | 1 | 11.401 |
| | KYKAKKRNYK | 2 | 10.361 |
| | KYKAKRKNYK | 3 | 11.271 |
| | KYKARKKNYK | 4 | 9.720 |
| | KYKAKRRNYK | 5 | 11.001 |
| | KYKARRKNYK | 6 | 9.897 |
| | KYKARRRNYK | 7 | 10.236 |
| | KYKARKRNYK | 8 | 11.120 |
| Group 2 | KYKAKKKSYK | 9 | 9.869 |
| | KYKAKKRSYK | 10 | 9.998 |
| | KYKAKRKSYK | 11 | 8.795 |
| | KYKARKKSYK | 12 | 7.997 |
| | KYKAKRRSYK | 13 | 9.237 |
| | KYKARRKSYK | 14 | 9.407 |
| | KYKARRRSYK | 15 | 8.991 |
| | KYKARKRSYK | 16 | 10.014 |
| Group 3 | KYRAKKKNYK | 17 | 9.400 |
| | KYRAKKRNYK | 18 | 9.161 |
| | KYRAKRKNYK | 19 | 8.999 |
| | KYRARKKNYK | 20 | 8.537 |
| | KYRAKRRNYK | 21 | 9.100 |
| | KYRARRKNYK | 22 | 7.997 |
| | KYRARRRNYK | 23 | 9.105 |
| | KYRARKRNYK | 24 | 9.333 |
| Group 4 | KYRAKKKSYK | 25 | 9.055 |
| | KYRAKKRSYK | 26 | 8.991 |
| | KYRAKRKSYK | 27 | 8.537 |
| | KYRARKKSYK | 28 | 9.198 |
| | KYRAKRRSYK | 29 | 9.525 |
| | KYRARRKSYK | 30 | 8.683 |
| | KYRARRRSYK | 31 | 9.100 |
| | KYRARKRSYK | 32 | 8.107 |
| Group 5 | KYAKKKKNYK | 33 | 7.709 |
| | KYAKKKRNYK | 34 | 7.031 |
| | KYAKKRKNYK | 35 | 7.527 |
| | KYAKRKKNYK | 36 | 6.999 |
| | KYAKKRRNYK | 37 | 7.032 |
| | KYAKRRKNYK | 38 | 7.777 |
| | KYAKRRRNYK | 39 | 6.989 |
| | KYAKRKRNYK | 40 | 7.635 |
| Group 6 | KYAKKKKSYK | 41 | 7.727 |
| | KYAKKKRSYK | 42 | 7.367 |
| | KYAKKRKSYK | 43 | 6.791 |
| | KYAKRKKSYK | 44 | 7.663 |
| | KYAKKRRSYK | 45 | 7.309 |
| | KYAKRRKSYK | 46 | 7.294 |
| | KYAKRRRSYK | 47 | 7.107 |
| | KYAKRKRSYK | 48 | 6.757 |
| Group 7 | KYARKKKNYK | 49 | 6.789 |
| | KYARKKRNYK | 50 | 6.109 |
| | KYARKRKNYK | 51 | 5.272 |
| | KYARRKKNYK | 52 | 5.991 |
| | KYARKRRNYK | 53 | 6.483 |
| | KYARRRKNYK | 54 | 6.531 |
| | KYARRRRNYK | 55 | 6.111 |
| | KYARRKRNYK | 56 | 5.798 |
| Group 8 | KYARKKKSYK | 57 | 6.059 |
| | KYARKKRSYK | 58 | 5.999 |
| | KYARKRKSYK | 59 | 5.510 |
| | KYARRKKSYK | 60 | 5.078 |
| | KYARKRRSYK | 61 | 5.787 |
| | KYARRRKSYK | 62 | 5.333 |
| | KYARRRRSYK | 63 | 5.727 |
| | KYARRKRSYK | 64 | 6.123 |

FIG. 2 shows the result of confirming the BSP and DMP1 gene expression by real-time PCR after treating the peptides of each group in human-derived mesenchymal stem cells in order to confirm the effect of the novel peptide on expression of BSP and DMP1 genes, which are marker genes for differentiation of osteoblasts and cementoblasts.

Peptides of all groups increased BSP gene expression by about 1.5 to 3 times and DMP1 gene expression by 6 to 10 times or more, compared to a control group. Particularly, peptides of group 1 showed the highest BSP and DMP1 mRNA expression values. Since the BSP and DMP1 genes are used as the marker genes for differentiation of osteoblasts and cementoblasts, and are known to be genes involved in the calcification process of bone and cementum, the peptide provided in the present invention was analyzed to have an effect of promoting bone and cementum regeneration.

FIG. 3 is a result of confirming expressions of DSP and BSP proteins by Western blot after treating human dental pulp cells with the novel peptide (SEQ ID NO: 1) in a concentration-dependent manner (1, 10, and 50 ug) to confirm the effect of novel peptides on expressions of marker proteins for differentiation of odontoblasts, osteoblasts and cementoblasts.

Referring to FIG. 3, it was confirmed that the expression of DSP which is a marker protein for differentiation of odontoblasts, and BSP which is a marker protein for differentiation of osteoblasts and cementoblasts, were increased in a concentration-dependent manner by the novel peptide.

FIG. 4 is the result of histological evaluation 3 weeks after application of 10 ug of the novel peptide (SEQ ID NO: 1) after damaging the dentine of beagle dog's teeth in order to confirm that the novel peptide induces physiological dentin regeneration in a animal model of damaged dentin.

Referring to FIG. 4, it was confirmed that there was no change in the lower part of the damaged dentin in the control group, but physiological dentin was formed in the lower part the damaged dentine in a test group treated with the novel peptide. These results mean that it is possible to treat pain caused by dental hyperesthesia, dentin dental caries, and odontoclasis caused by damaged dentin through physiological dentin regeneration.

Based on the in vitro test results shown in FIG. 2 and Tables 10 and 11, in order to confirm the effect of the peptide of Group 1 (SEQ ID NO: 1) on hard tissue formation in vivo, FIG. 5 shows the result confirming that SEQ ID NO: 1 peptide (10 ug) of group 1 or human dental pulp cells (hDPCs) of control (cell only) were transplanted into the subcutaneous tissue of mice with impaired immune systems, and the proportion of hard tissue formation was increased in the group treated with the novel peptide compared to the control group 12 weeks after transplantation (A-D: hDPCs-only; E-H: group 1 peptide; I-L: BMP2 treatment/size bar; A, E, and I: 500 µm; B, F, J: 200 µm; C, G, and K: 100 µm; D, H and L: 50 µm)

Referring to FIG. 5, in a histological analysis through hematoxylin-eosin staining, it was observed that a bone/cementum-like tissue incorporating cells into matrix of a newly formed calcified tissue around HA/TCP particles in the group treated with hDPCs-alone, peptide of group 1, and positive control BMP2 was formed. However, a test group treated with the novel peptide showed more similar hard tissue formation as the BMP-2 treated group compared to the control group. Taken together, the results indicate that the novel peptide used in this experiment exhibits an effect capable of promoting the regeneration of bone/cementum-like tissue and dentin/pulp tissue complex.

In order to confirm the effect of Group 1 (SEQ ID NO: 1) peptides on periodontal ligament formation in vivo, FIG. 6 shows the result confirming that SEQ ID NO: 1 peptide (10 ug) of group 1 or human dental pulp cells (hDPCs) of control (cell only) were transplanted into the subcutaneous tissue of mice with impaired immune systems, and compared to the control group 6 weeks after transplantation (size bar; A, C: 200 µm; B and D: 100 µm).

Referring to FIG. 6, it was confirmed that a periodontal ligament fiber bundle (black triangle) were formed in the group treated with the novel peptide, compared to the control group 6 weeks after transplantation. Specifically, in the histological analysis through hematoxylin-eosin staining, it was observed that irregular fiber arrangement was observed in hDPCs-alone, but in a group treated with novel peptides of group 1, the periodontal ligament-like tissue incorporating the fiber bundle was formed as a newly formed calcified tissue around the HA/TCP particle. The above results suggest that the novel peptide used in this experiment may have an effect of promoting regeneration of damaged periodontal ligament.

This study was conducted as part of the national research and development project (1465037227) titled "Research and Development of Medical Technologies in Dentistry" by the Korea Health Industry Development Institute in 2022, with the aim of developing a chronic periodontitis treatment technique using bio-convergence novel material CPNE7 peptide.

In the present specification and drawings, preferred embodiments of the present invention have been disclosed, and although specific terms are used, these are only used in a general sense to easily explain the technical content of the present invention and help the understanding of the present invention. It is not intended to limit the scope. It will be apparent to those skilled in the art that other modifications based on the technical concept of the present invention can be performed in addition to the embodiments disclosed herein.

## Claims

1. A peptide for promoting regeneration of a hard tissue or a dental pulp tissue and treating a dentin-dental pulp disease or a periodontal disease, wherein the peptide consists of any one of amino acid sequences of SEQ ID NOs: 1 to 8.

2. The peptide of claim 1, wherein the peptide is subjected to N-terminal or C-terminal acetylation, or amidation.

3. The peptide of claim 1, wherein the hard tissue comprises dentin, bone, and cementum.

4. A polynucleotide encoding the peptide of any one of claims 1 to 3.

5. An expression vector comprising the polynucleotide of claim 4.

6. A pharmaceutical composition for preventing or treating a dentin-dental pulp disease, comprising the peptide of any one of claims 1 to 3.

7. The pharmaceutical composition of claim 6, wherein the dentin-dental pulp disease is dentine hyperesthesia, pulp hyperemia, pulpitis, pulp modification, pulp necrosis, and gangrene.

8. A pharmaceutical composition for preventing or treating a periodontal disease, comprising the peptide of any one of claims 1 to 3.

9. The pharmaceutical composition of claim 8, wherein the periodontal disease is gingivitis, periodontitis, periodontal pocket, or periodontal abscess.

10. A quasi-drug composition for preventing or improving a dentin-dental pulp disease or a periodontal disease, comprising the peptide of any one of claims 1 to 3.

11. A health functional food composition for preventing or improving a dentin-dental pulp disease or a periodontal disease, comprising the peptide of any one of claims 1 to 3.

12. A method for preventing or treating a dentin-dental pulp disease in a non-human subject, comprising administering a composition including the peptide of any one of claims 1 to 3 to the subject.

13. A method for preventing or treating a periodontal disease in a non-human subject, comprising administering a composition including the peptide of any one of claims 1 to 3 to the subject.

14. A method for promoting regeneration of a hard tissue including dentin, bone and cementum, or a dental pulp tissue in a non-human subject, comprising administering a composition including the peptide of any one of claims 1 to 3 to the subject.

15. The peptide of claim 1, which is for promoting regeneration of a hard tissue or a dental pulp tissue and treating a dentin-dental pulp disease or a periodontal disease, wherein the peptide consists of any one of amino acid sequences of SEQ ID NOs: 1 to 8.
